**Europäisches Patentamt**

**European Patent Office**

**Office européen des brevets**

(19)

(11) Veröffentlichungsnummer: **0 149 857 B1**

(12) # EUROPÄISCHE PATENTSCHRIFT

(45) Veröffentlichungstag der Patentschrift:
20.04.88

(51) Int. Cl.⁴: **C 07 D 213/807**

(21) Anmeldenummer: **84201443.3**

(22) Anmeldetag: **09.10.84**

(54) Verfahren zur Herstellung von Chinolinsäure aus Chinolin.

(30) Priorität: **14.12.83 DE 3345223**

(43) Veröffentlichungstag der Anmeldung:
**31.07.85 Patentblatt 85/31**

(45) Bekanntmachung des Hinweises auf die Patenterteilung:
**20.04.88 Patentblatt 88/16**

(84) Benannte Vertragsstaaten:
**CH DE FR GB IT LI NL**

(56) Entgegenhaltungen:
**EP - A - 0 024 197**
**EP - A - 0 034 943**
**DE - A - 3 150 005**
**DE - C - 945 147**
**US - A - 2 371 691**
**US - A - 2 586 555**

**Die Akte enthält technische Angaben, die nach dem Eingang der Anmeldung eingereicht wurden und die nicht in dieser Patentschrift enthalten sind.**

(73) Patentinhaber: **RÜTGERSWERKE AKTIENGESELLSCHAFT, Mainzer Landstrasse 217, D-6000 Frankfurt am Main 1 (DE)**

(72) Erfinder: **Orth, Winfried, Dr., Am Schachtelgraben 28, D-6733 Hassloch/Pfalz (DE)**
Erfinder: **Pastorek, Emmerich, Dr., Grünbergerstrasse 90, D-6944 Hemsbach (DE)**
Erfinder: **Fickert, Werner, Dr., Stockacher Strasse 14, D-6800 Mannheim (DE)**

## Beschreibung

Die Erfindung betrifft ein neues, verbessertes Verfahren zur Herstellung von Pyridin-2,3-dicarbonsäure. Diese Verbindung wird allgemein als Chinolinsäure bezeichnet.

Chinolinsäure wird zur Herstellung pharmazeutisch wirksamer Verbindungen verwendet, wie z.B. Lokalanästhetika, Bakteriziden oder Verbindungen, die gegen Stoffwechselstörungen eingesetzt werden können.

Aus der Literatur sind verschiedene Verfahren zur Herstellung von Chinolinsäure bekannt. Sie beruhen teils auf der Oxidation von Chinolin und teils auf der Oxidation von am aromatischen Kern aktivierend substituierten Chinolinderivaten.

Die zuerst von Hoogewerff und van Dorp (Ber. dtsch. chem. Ges., Abt. B, 12, 747, (1879) beschriebene Methode der Oxidation von Chinolin und Kaliumpermanganat in alkalischem Milieu erbringt nur sehr geringe Ausbeuten an Chinolinsäure neben einer grossen Menge an anderen Reaktionsprodukten.

Die weiteren Verfahren zur Oxidation von Chinolin beruhen im wesentlichen auf der von Stix und Bulgatsch (Ber. dtsch. chem. Ges., 65, (Abt. B) 11 (1932) beschriebenen Methode der Oxidation mit Wasserstoffperoxid in Anwesenheit eines Kupfersalzes. Da diese Reaktion recht schwierig zu handhaben ist, wurden in der Folgezeit Modifizierungen entwickelt, die eine bessere Steuerung der Reaktion und eine leichte Erhöhung der Ausbeute bedingen. Derartige Änderungen sind z.B. in der EP-A-0 024 197 oder EP-A-0 034 943 offenbart. Bei all diesen Verfahren entstehen zuerst Kupfersalze der Chinolinsäure, aus denen die freie Säure mit Hilfe eines Sulfids freigesetzt werden muss. Abgesehen davon, dass dies ein weiterer, unerwünschter Reaktionsschritt ist, zeigt es sich, dass die vollständige Abtrennung der Kupferionen äusserst schwierig ist, so dass die entsprechend hergestellten Chinolinsäuren immer Spuren von Kupfer enthalten.

Aus DE-A-31 50 005 ist ein einfaches, umweltfreundliches Verfahren zur Herstellung von Chinolinsäure in hoher Reinheit und guter Ausbeute bekannt, bei dem Chinolinderivate mit Chlorationen oxidiert werden, wobei Vanadyl(V)-Kationen als Katalysator dienen.

Der Nachteil dieses Verfahrens ist es, dass lediglich solche Chinolinderivate zu Chinolinsäure oxidiert werden, in denen am Benzolkern mindestens ein Wasserstoffatom durch eine aktivierende Gruppe substituiert ist, während das leicht zugängliche und preiswerte unsubstituierte Chinolin in diesem Verfahren nicht oxidiert werden kann.

Es bestand daher die Aufgabe, ein neues einfaches, wirtschaftliches und umweltfreundliches Verfahren zu finden, bei dem sich Chinolinsäure in hoher Reinheit aus unsubstituiertem Chinolin herstellen lässt.

Die Lösung der Aufgabe erfolgt durch ein Verfahren gemäss den Ansprüche 1 bis 4.

Es wurde gefunden, dass Chinolin in saurem, wässrigem Milieu in Gegenwart katalytischer Mengen von Cobalt(III)-, Titanyl(IV)-, Vanadyl(V)- oder Osmium(VIII)-Kationen mit Wasserstoffperoxid reagiert unter Bildung wasser-löslicher Oxidationsprodukte. Diese Oxidationsreaktion ist nicht einheitlich und nicht exakt reproduzierbar. Dementsprechend entstehen auch keine definierten Oxidationsprodukte.

Wird diese Oxidation durchgeführt, bis kein Wasserstoffperoxid mehr verbraucht wird, eine Reaktion, bei der wesentlich mehr als die theoretisch zur Bildung der Dicarbonsäure stöchiometrisch notwendige Menge an Wasserstoffperoxid benötigt wird, so entsteht ein nicht aufarbeitbares Gemisch aus hydroxyl- und carboxylendständigen Bruchstücken aus dem aromatischen und dem Pyridinring des Chinolins.

Es wurde nun gefunden, dass sich diese Reaktion im Sinne einer bevorzugten Bildung von Chinolinsäure steuern lässt, wenn man Wasserstoffperoxid voroxidiert und danach die Oxidation zur Chinolinsäure mit Chlorit- oder Chlorationen durchführt. Dabei erfolgt wahrscheinlich im Reaktionsschritt der Voroxidation eine Aktivierung und/oder eine Aufspaltung des aromatischen Rings und in der zweiten Oxidationsstufe eine gezielte Oxidation der aktivierten Produkte zu Chinolinsäure.

Die Menge des erfindungsgemäss einzusetzenden Wasserstoffperoxids muss dabei geringer sein als die Wasserstoffperoxidmenge, die bei einer vollständigen Reaktion mit Chinolin verbraucht wird. Sie ist zweckmässigerweise niedriger als die stöchiometrisch zur Herstellung der Pyridincarbonsäure notwendige Menge und liegt bei 20 bis 90%, bevorzugt bei 30 bis 80%.

Bereits mit 20% der stöchiometrisch notwendigen Wasserstoffperoxidmenge erhält man nach der Chlorit- oder Chloratoxidation eine Ausbeute an Chinolinsäure von 44%, bezogen auf das eingesetzte Chinolin. Das nicht umgesetzte Chinolin kann dabei zurückgewonnen werden. Mit steigender Wasserstoffperoxidmenge bei der Voroxidation steigt die Ausbeute an Chinolinsäure, bis sie bei 80% der stöchiometrisch notwendigen Menge an Wasserstoffperoxid mit 51 bis 52%, bezogen auf das eingesetzte Chinolin, ein Maximum erreicht.

Gleichzeitig aber steigt auch die Menge der entstehenden unerwünschten, nicht definierten Oxidationsprodukte, die nicht mehr in Chinolinsäure umgewandelt werden können, so dass z.B. ab 90% der stöchiometrisch notwendigen Wasserstoffperoxidmenge ein starker Abfall der Chinolinsäureausbeute resultiert. Als Wasserstoffperoxid können sowohl das reine Wasserstoffperoxid in Form seiner wässrigen Lösungen als auch seine Additionsverbindungen wie Alkalicarbonat-, Alkaliborat-, Phosphat- oder Harnstoff-peroxyhydrat eingesetzt werden.

Das zweite Oxidationsmittel, mit dem die voroxidierten, aktivierten Produkte zu Chinolinsäure oxidiert werden, sind Verbindungen aus der Gruppe der Sauerstoffverbindungen des Chlors. Als Quelle können alle wasserlöslichen Chlorite oder Chlorate eingesetzt werden. Vorzugsweise werden jedoch die Chlorate der Erdalkali- und Alkalimetalle und des Ammoniums verwendet. Insbesondere eignet sich das gut handhabbare, kristallwasserfreie, aber gut wasserlösliche Natriumchlorat.

Die Menge an einzusetzendem Chlorit oder Chlorat ist umgekehrt proportional zur verwendeten Wasserstoffperoxidmenge. Um eine gute Ausbeute und ein schnelles Beenden der Oxidationsreaktion zu erzielen, ist es angebracht, mit einem Überschuss an Oxidationsmittel (= Summe aus Wasserstoffperoxid und Chlorit oder Chlorat) zu arbeiten. Es wurde gefunden, dass mit 120 bis 150%, bevorzugt mit 130% der theoretisch zur oxidativen Herstellung von Chinolinsäure aus Chinolin notwendigen Menge an Oxidationsmittel die beste Wirtschaftlichkeit des erfindungsgemässen Verfahrens gegeben ist.

Als Katalysator für beide Oxidationsreaktionen dienen sowohl Vanadyl(V)- als auch Cobalt(II)-, Titanyl(IV)- oder Osmium(VIII)-Kationen, die durch Auflösen entsprechender wasserlöslicher Salze im sauren Reaktionsgemisch bereitgestellt werden. Die Menge an Katalysator, die pro Reaktionsansatz eingesetzt wird, liegt zwischen 0,01 und 0,1 g Salz pro Mol Chinolin, wobei naturgemäss eine höhere Katalysatormenge zu einer grösseren Reaktionsgeschwindigkeit führt.

Die Reaktion wird in saurem, wässrigem Medium bei Temperaturen im Bereich von 50 bis 100 °C durchgeführt. Als Säuren eignen sich insbesondere Mineralsäuren wie Salz-, Salpeter-, Phosphor- oder bevorzugt Schwefelsäure. Die Aufarbeitung des Reaktionsgemisches erfolgt nach an sich bekannten Verfahren.

Beispiel

In einer Mischung aus 1,6 l Wasser und 0,45 l konzentrierter Schwefelsäure werden 516 g (4 Mol) Chinolin und 0,2 g Ammoniumvanadat gelöst. Die Lösung wird auf 65–70° erhitzt und bei dieser Temperatur innerhalb von 3–4 h mit 406 ml (10,8 Mol) Wasserstoffperoxid, 70%-ig, versetzt. Die Reaktion ist leicht exotherm. Um die Reaktionstemperatur während der Wasserstoffperoxidzugabe bei 70 °C zu halten, genügt es, das Wasserbad auf ca. 60 °C zu temperieren. Nach der Zugabe des Oxidationsmittels wird noch ca. 3 h bei 70–75 °C nachgerührt. Nach dieser Zeit ist das Wasserstoffperoxid verbraucht (Kontrolle mit Jod-Stärke-Papier!). Danach wird das Reaktionsgemisch auf 85–90 °C erhitzt und bei 90–100 °C während 3 h mit einer Lösung aus 1384 g (15 Mol) Natriumchlorat und 2 l Wasser versetzt. Die Reaktion mit Natriumchlorat ist anfangs stark exotherm. Das Reaktionsgemisch muss daher gekühlt werden. Die anfangs helle Reaktionslösung wird während der Oxidation mit Wasserstoffperoxid dunkler und mit Natriumchlorat noch dunkler. Erst zum Schluss der Oxidation wird das Reaktionsgemisch hellgelb. Schon bei der Oxidation mit Wasserstoffperoxid entweicht aus der Reaktionslösung $CO_2$. Die Gasentwicklung nimmt bei Anwendung von Natriumchlorat in der zweiten Stufe zu. Zum Schluss der Oxidation kann sich explosives Chlordioxid bilden. Darum werden die Abgase in einem Absorptionsgefäss aufgefangen, in welchem 50 g Natriumhydrogensulfit in 0,5 l Wasser gelöst und mit 10 g Magnesiumoxid versetzt werden. Nach Zugabe des Oxidationsmittels wird noch 3 h bei 95 °C nachgerührt. Zum Schluss der Chloratzugabe und während der Nachreaktionszeit muss die Apparatur mit $CO_2$ gespült werden, um etwa auftretende Chlordioxidgase zu verdünnen. Nach Abkühlen des Reaktionsgemisches auf ca. 80 °C werden zu diesem vorsichtig 910 ml Natronlauge, 50%-ig, langsam zugegeben, bis ein pH-Wert von 8,5 eingestellt ist. Es scheidet sich das nicht verbrauchte Chinolin ab, das bei 40–50 °C in 100 ml Toluol aufgenommen wird. Es werden 80 g Chinolin isoliert und für den nächsten Ansatz verwendet. Das Reaktionsgemisch wird mit 195 ml konz. Salzsäure auf pH 4,5 eingestellt und mit 30 g Aktivkohle versetzt. Dieses Gemisch wird ca. 15 min gerührt, bei ca. 60 °C abgenutscht und dreimal mit je 100 ml 50 °C warmem Wasser nachgewaschen. Das erhaltene Filtrat wird bei 50 °C mit 640 ml konz. Salzsäure auf pH 1 gebracht und auf 0 °C abgekühlt. Das ausgefallene Produkt wird abgenutscht, mit 400 ml kaltem Wasser sulfatfrei gewaschen und bei 80–105 °C getrocknet.

Ausbeute: 346 g Chinolinsäure entsprechend 52% der Theorie, bezogen auf das eingesetzte Chinolin. Die Chinolinsäure hat einen Aschegehalt von 0,1% und eine Säurezahl von 667 (theoretisch 671).

Aus der Mutterlauge können durch Fällen mit Kupfer(II)-ionen noch geringe Mengen an Chinolinsäure abgetrennt werden, die aber aufgrund schwer zu entfernender Reste an Kupferionen nicht der durch Ansäuern ausgefällten Chinolinsäure zugeschlagen werden dürfen, insbesondere dann nicht, wenn die Chinolinsäure zu pharmazeutischen Präparaten weiter verarbeitet werden soll.

**Patentansprüche**

1. Verfahren zur Herstellung von Chinolinsäure durch Oxidation von Chinolin in saurem, wässrigem Milieu, dadurch gekennzeichnet, dass Chinolin in Gegenwart von Vanadyl(V)-, Cobalt(II)-, Titanyl(IV)- oder Osmium(VIII)-kationen in einer Menge zwischen 0,01 bis 0,1 g Salz pro Mol Chinolin bei Temperaturen im Bereich von 50 bis 100 °C zuerst mit Wasserstoffperoxid voroxidiert und anschliessend mit Chlorit- oder Chlorationen zu Chinolinsäure oxidiert wird.

2. Verfahren nach Anspruch 1, dadurch gekennzeichnet, dass Chinolin mit 20 bis 90% der stöchiometrisch notwendigen Menge an Wasserstoffperoxid voroxidiert wird.

3. Verfahren nach Anspruch 1 und 2, dadurch gekennzeichnet, dass Chinolin mit 30 bis 80% der stöchiometrisch notwendigen Menge an Wasserstoffperoxid voroxidiert wird.

4. Verfahren nach Anspruch 1 bis 3, dadurch gekennzeichnet, dass die Summe der verwendeten Oxidationsmittel 120 bis 150% der stöchiometrisch notwendigen Menge beträgt.

**Revendications**

1. Procédé de préparation de l'acide quinoléique par oxydation de quinoléine en milieu acide aqueux, caractérisé par le fait que de la quinoléine est tout d'abord préoxydée à l'aide d'eau oxygénée en présence de cations vanadyl(V), cobalt (II), titanyl(IV) ou osmium(VIII) en une quantité située entre 0,01 et 0,1 g de sel par mole de quinoléine à des températures situées dans le domaine de 50 à 100 °C et ensuite oxydée en acide quinoléique à l'aide d'ions chlorite ou chlorate.

2. Procédé selon la revendication 1, caractérisé par le fait que la quinoléine est préoxydée à l'aide de 20 à 90% de la quantité stœchiométriquement nécessaire d'eau oxygénée.

3. Procédé selon la revendication 1 et 2, caractérisé par le fait que la quinoléine est préoxydée à l'aide de 30 à 80% de la quantité stœchiométriquement nécessaire d'eau oxygénée.

4. Procédé selon la revendication 1 à 3, caractérisé par le fait que la somme des agents oxydants utilisés représente 120 à 150% de la quantité stœchiométriquement nécessaire.

**Claims**

1. A process for the preparation of quinolinic acid by oxidation from quinoline in an acid, aqueous medium, characterized in that quinoline is first previously oxidized with hydrogen peroxide in the presence of vanadyl(V), cobalt(II), titanyl(IV) or osmium(VIII) cations in a quantity of between 0,01 and 0,1 g salt per mol of quinoline at temperatures in the range of 50 to 100 °C and is then oxidized with chlorite or chlorate ions to form quinolinic acid.

2. A process according to Claim 1, characterized in that quinoline is previously oxidized with from 20 to 90% of the stoichiometrically necessary quantity of hydrogen peroxide.

3. A process according to Claims 1 and 2, characterized in that quinoline is previously oxidized with from 30 to 80% of the stoichiometrically necessary quantity of hydrogen peroxide.

4. A process according to Claims 1 to 3, characterized in that the sum of the oxidation agents used amounts to from 120 to 150% of the stoichiometrically necessary quantity.